# EUROPEAN PATENT APPLICATION

(11) **EP 4 600 967 A1**
(43) Date of publication of application: **13.08.2025**
(21) Application number: 24156475.6
(22) Date of filing: 08.02.2024
(51) Int. Cl.: G16H 20/40, G06V 10/82, G06V 20/40, G16H 40/20, A61B 34/20

(54) **PROVIDING ANNOTATED DATA FOR TRAINING A MACHINE LEARNING MODEL TO MONITOR EVENTS DURING A MEDICAL PROCEDURE**

(71) Applicant: Koninklijke Philips N.V., 5656 AG Eindhoven (NL)
(72) Inventor: NETO FONSECA, Lucia Teresa, Eindhoven (NL); HENDRIKS, Bernardus Hendrikus Wilhelmus, Eindhoven (NL); KESSELS, Angelique Carin Johanna Maria, 5656AG Eindhoven (NL); BACHVAROV, Chavdar Ludmilov, Eindhoven (NL); BOON, Sjirk Niels, Eindhoven (NL); VAN RIEL, Sjors, Eindhoven (NL)
(74) Representative: Philips Intellectual Property & Standards

(57) **Abstract**

A computer-implemented method of providing annotated data is provided. The method includes: receiving video data (110), the video data capturing events during a plurality of instances of a medical procedure. The method also include receiving sensor data (120) representing the events during the instances of the medical procedure. The sensor data (120) for each instance of the medical procedure is generated contemporaneously with the video data (110) for each instance of the medical procedure. The method also includes assigning labels (130_{1..i}) to the events captured in the video data (110) using the sensor data (120), and outputting the video data (110) and the labels (130_{1..i}) to provide the annotated data (140).

## Description

### TECHNICAL FIELD

The present disclosure relates to providing annotated data. The annotated data may be used to train a machine learning model to monitor events during a medical procedure. A computer-implemented method of providing annotated data, a computer-implemented method of training a machine learning model to monitor events during a medical procedure, and a computer-implemented method of monitoring events during a medical procedure, are provided. A computer program product, and also a system, that correspond to each of the methods, are also provided.

### BACKGROUND

Medical procedures are routinely monitored in order to assess their progress. In case of deviations from a scheduled workflow, additional resources may be introduced in order to remedy the situation. For instance, if a procedure is taking longer than expected, or if complications are encountered, additional medical professionals, or additional equipment, may be brought-in to support the procedure. The monitoring is typically performed manually, for example by a medical professional, or by another person observing the procedure.

Various events may take place in medical procedures, and the progress is assessed by monitoring the times, and also durations, of these events in relation to the scheduled workflow. For instance, a cardiac catheterization procedure may begin with a medical professional, such as a physician, or a nurse, entering the cath lab. The patient is then brought into the cath lab. Various vital sign monitoring equipment is then attached to the patient. An access site, for instance in the arm, or in leg, may then be cleansed, and the patient is then covered with sterile drapes. The patient may then be sedated. A needle may then be injected into the access site. A guidewire may then be inserted into the needle and navigated to the treatment side under the guidance of projection X-ray imaging. The needle is then removed, and a sheath is inserted over the guidewire. The guidewire is then removed and the catheter is inserted through the sheath to a cardiac treatment site. When the catheter is correctly positioned, a contrast agent may be injected into the patient, and the vasculature imaged using projection X-ray imaging. An intervention, such as angioplasty, stenting, heart valve repair, and so forth, may then be performed at the treatment site under the guidance of live projection X-ray imaging. When the intervention has been completed, the catheter, sheath, and guidewire, are removed, and a pressure dressing, or a vascular closure device is applied in order to close the access site. The patient is then transferred to a recovery room.

The manual monitoring of such events during medical procedures is both challenging, and demanding, on an observer. Consequently, it has been proposed to automate some of these aspects by using machine learning-based models. However, the training of such models requires a significant amount of annotated data. The annotation of the data is typically performed manually. This is a tedious process, and consequently it presents a bottleneck in the adoption of automated approaches.

Thus, there remains a need to improve the annotation of data that is used to train machine learning models to monitor events during medical procedures.

### SUMMARY

According to a first aspect of the present disclosure, a computer-implemented method of providing annotated data, is proposed. The method comprises:
receiving video data, the video data capturing events during a plurality of instances of a medical procedure;
receiving sensor data, the sensor data representing the events during the instances of the medical procedure, and wherein the sensor data for each instance of the medical procedure is generated contemporaneously with the video data for each instance of the medical procedure;
assigning labels to the events captured in the video data using the sensor data; and
outputting the video data and the labels to provide the annotated data.

In the above method, labels are assigned to events captured in the video data using simultaneously-generated sensor data. Examples of labels that may be applied to the video data include "nurse enters the cath lab", "guidewire insertion initiated", "X-ray imaging procedure initiated", "access site closure with vascular access device in progress", and so forth. Since the labels are assigned to the video data using the sensor data, the burden of manually labelling the video data is reduced. This reduces the burden of providing annotated data for training a machine learning algorithm to monitor events during medical procedures.

According to a second aspect of the present disclosure, a computer-implemented method of monitoring events captured in one or medical images generated by a medical imaging system during a medical imaging procedure, is proposed. The method includes:
receiving medical imaging system log file data generated by the medical imaging system during the medical imaging procedure;
inputting the medical imaging system log file data into a trained machine learning model, the trained machine learning model being trained to predict, from the log file data, one or more events captured in the one or more medical images generated during the medical procedure;
predicting, using the trained machine learning model, and based on the inputted medical imaging system log file data, one or more events captured in the one or more medical images generated during the medical procedure; and
outputting the predicted one or more events; and
wherein the machine learning model is trained to predict the one or more events captured in the one or more medical images generated during the medical procedure using annotated data, the annotated data comprising medical imaging system log file data generated during a plurality of instances of the medical imaging procedure, and ground truth event labels representing one or more events captured in one or more medical images generated during the instances of the medical procedure, and wherein the ground truth event labels are extracted from the one or more medical images.

In this method, a machine learning model is trained to predict events captured in medical images generated during a medical procedure. Examples of events that may be predicted in accordance with this second aspect include "catheter positioned in treatment site" "angioplasty balloon inflation in progress", "stent expansion initiated", and so forth. The events are predicted from medical imaging system log file data. Thus, at inference, the events are predicted without the need to analyze the medical images themselves. This circumvents privacy issues that might otherwise be encountered by determining the events by analyzing the medical images. The machine learning model that is used in this method is trained to predict the events using annotated data that comprises medical imaging system log file data that is generated during a plurality of instances of the medical imaging procedure, and ground truth event labels. The ground truth event labels are extracted from the one or more medical images that are generated during the instances of the medical procedure. This is in contrast to the method described in accordance with the first aspect and wherein the annotated data included video data that was labelled with event labels that were assigned using sensor data. Since in this method the event labels are extracted from medical images, accurate event labels are provided, and the burden of manually labelling the log file data is also reduced.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a computer-implemented method of providing annotated data, in accordance with some aspects of the present disclosure.
Fig. 2 is a schematic diagram illustrating an example of a system 200 for providing annotated data, in accordance with some aspects of the present disclosure.
Fig. 3 is a schematic diagram illustrating an example of various elements of a computer-implemented method of providing annotated data, in accordance with some aspects of the present disclosure.
Fig. 4 is a schematic diagram illustrating an example of various elements of a computer-implemented method of training a machine learning model 320 to monitor events during a medical procedure, in accordance with some aspects of the present disclosure.
Fig. 5 is a schematic diagram illustrating an example of various elements of a computer-implemented method of monitoring events during a medical procedure, in accordance with some aspects of the present disclosure.
Fig. 6 is a schematic diagram illustrating an example of a system 400 for monitoring events during a medical procedure, in accordance with some aspects of the present disclosure.
Fig. 7 is a flowchart illustrating an example of a computer-implemented method of monitoring events captured in one or medical images 510 generated by a medical imaging system 150 during a medical imaging procedure, in accordance with some aspects of the present disclosure.
Fig. 8 is a schematic diagram illustrating an example of a system 500 for monitoring events captured in one or medical images 510 generated by a medical imaging system 150 during a medical imaging procedure, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a corresponding computer program product, and in a corresponding system, in a corresponding manner.

In the following description, reference is made to computer-implemented methods that relate to the provision of annotated data. The annotated data may be used to train a machine learning model to monitor events during a medical procedure. In some examples, reference is made to examples of medical procedures that are performed in a cath lab. For instance, reference is made to an angioplasty procedure. It is, however, to be appreciated that the cath lab and the angioplasty serve only as examples, and that the methods may be used more generally to provide training data for training machine learning models to monitor events during medical procedures in general. These include diagnostic and also treatment procedures. By way of some examples, it is contemplated that the methods may be used to provide training data for training machine learning models to monitor events during medical procedures such as cardiac procedures, and orthopaedic procedures.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed in the computer-implemented methods disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations performed in the computer-implemented methods disclosed herein.

As mentioned above, there remains a need to improve the annotation of data that is used to train machine learning models to monitor events during medical procedures.

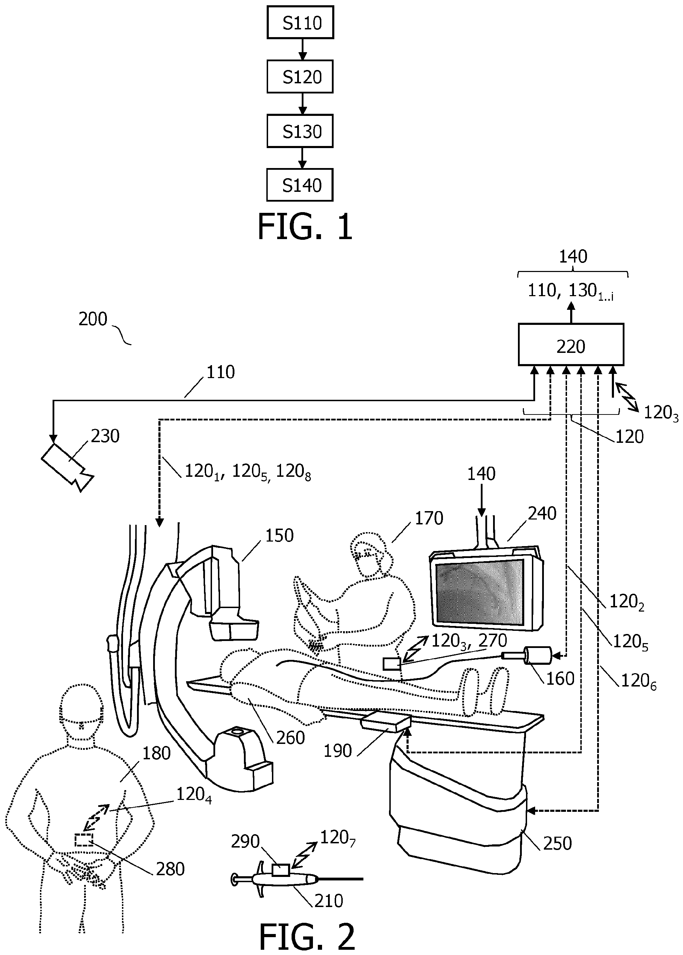

Fig. 1 is a flowchart illustrating an example of a computer-implemented method of providing annotated data, in accordance with some aspects of the present disclosure. Fig. 2 is a schematic diagram illustrating an example of a system 200 for providing annotated data, in accordance with some aspects of the present disclosure. It is noted that operations that are described in relation to the method illustrated in Fig. 1, may also be performed by the one or more processors 220 of the system 200 illustrated in Fig. 2. Likewise, operations that are described as being performed by the one or more processors 220 of the system 200 illustrated in Fig. 2, may also be performed in the method described with reference to Fig. 1. With reference to Fig. 1, and Fig. 2, the computer-implemented method of providing annotated data, includes:
receiving S110 video data 110, the video data capturing events during a plurality of instances of a medical procedure;
receiving S120 sensor data 120, the sensor data representing the events during the instances of the medical procedure, and wherein the sensor data 120 for each instance of the medical procedure is generated contemporaneously with the video data 110 for each instance of the medical procedure;
assigning S130 labels 130_{1..i} to the events captured in the video data 110 using the sensor data 120; and
outputting S140 the video data 110 and the labels 130_{1..i} to provide the annotated data 140.

In the above method, labels are assigned to events captured in the video data using simultaneously-generated sensor data. Examples of labels that may be applied to the video data include "nurse enters the cath lab", "guidewire insertion initiated", "X-ray imaging procedure initiated", "access site closure with vascular access device in progress", and so forth. Since such labels are assigned to the video data using the sensor data, the burden of manually labelling the video data is reduced. This reduces the burden of providing annotated data for training a machine learning algorithm to monitor events during medical procedures.

The operations that are performed in the method illustrated in Fig. 1 are described in more detail below.
Referring initially to the operation S110; in this operation, video data 110 is received. The video data 110 captures events during a plurality of instances of a medical procedure.

The medical procedure that is captured in the video data 110 may in general be any type of medical procedure. Examples of medical procedures that may be captured in the video data 110 include cardiac procedures such as an angioplasty procedure, or a stenting procedure, or a heart valve repair procedure.

The events that are captured in the video data 110 may generally be referred-to as workflow events. For instance, in the example of an angioplasty procedure, events such as the following may be captured: a medical professional such as a physician, or a nurse entering, the cath lab; a patient being brought into the cath lab; the attaching of various vital sign monitoring equipment to the patient; the cleansing of an access site, for instance in the arm, or in leg; the covering of the patient with sterile drapes; the sedation of the patient; the injection of a needle into the access site; the insertion of a guidewire into the needle; the navigation of the guidewire to the treatment side under the guidance of projection X-ray imaging; the removal of the needle; the insertion of a sheath over the guidewire; the removal of the guidewire; the insertion of the catheter through the sheath to a cardiac treatment site; the injection of a contrast agent into the patient; the imaging of the vasculature using projection X-ray imaging; the performance of the angioplasty procedure at the treatment site under the guidance of live projection X-ray imaging; the removal of the catheter, sheath, and guidewire; the application of a pressure dressing, or a vascular closure device to close the access site; and the transfer of a patient to a recovery room.

The video data that is received in the operation S 110 may capture events such as those mentioned above in some tens, or hundreds, or more, medical procedures. The procedures may be performed by a range of different medical professionals, and with patients having different ages, gender, body mass index, co-morbidities, and so forth.

An example of the capture of video data 110 is illustrated in Fig. 2, and wherein the video data 110 captures events that are performed by a nurse 170 and a physician 180 during an angioplasty procedure. In the illustrated example, the nurse is covering a patient 260 with sterile drapes whilst the physician 180 is in attendance. In the illustrated example, the video data is generated by a camera 230.

In some examples, the video data 110 that is received in the operation S 110 is generated by a camera that is responsive to optical radiation. For instance, in one example, the video data is generated by a camera that is responsive to optical radiation within the visible spectrum. The video data may be generated by a so-called "RGB", i.e. Red-Green-Blue, camera, that is responsive to red, green, and blue wavelengths, for example. In another example, the video data is generated by an infrared camera that is responsive to optical radiation within the infrared spectrum. In another example, the video data may be generated by a hyperspectral camera. In another example the video data is generated by a so-called depth camera that is responsive to a range between the camera and objects within a field of view of the camera. Depth cameras may determine the range based on principles that employ time-of-flight "TOF", LIDAR, structured light, binocular stereo vision, and so forth. Examples of depth cameras include the Azure Kinect DK depth camera, and the Intel RealSense^{™} LiDAR Camera L515. In another example, the video data is generated by a medical imaging system. For instance, the video data may be generated by a projection X-ray medical imaging system, or a computed tomography "CT", or an magnetic resonance imaging "MRI" system, or by an ultrasound imaging system. Such imaging systems may generate video data in the form of a temporal sequence of images. For instance, a projection X-ray medical imaging system may operate in a so-called fluoroscopy mode in which a temporal sequence of images is generated. In yet another example, the video data 110 that is received in the operation S110 includes a combination of two of more of the aforementioned sources of video data.

The video data 110 that is received in the operation S 110 may be received via any form of data communication, including via wired, or wireless, or optical fiber communication. By way of some examples, when wired data communication is used, the communication may take place via electrical signals that are transmitted on an electrical cable. When wireless data communication is used, the communication may take place via RF or infrared signals. When an optical fiber data communication is used, the communication takes place via optical signals that are transmitted on an optical fiber.
Referring now to the operation S120 illustrated in Fig. 1; in this operation, sensor data 120 is received. The sensor data 120 represents the events during the instances of the medical procedure. The sensor data 120 for each instance of the medical procedure is generated contemporaneously with the video data 110 for each instance of the medical procedure.

Various types of sensor data may be received in the operation S120, some examples of which are illustrated in Fig. 2. The sensor data 120 may include timestamps corresponding to the time of its acquisition. By way of some examples, the sensor data may include one or more of: log data 120₁, 120₂ generated by a medical device 150, 160 captured in the video data 110, location data 120₃, 120₄ representing a location of one or more medical professionals 170, 180 captured in the video data 110, motion data representing a motion of one or more medical professionals 170, 180 captured in the video data, medical image data generated by a medical imaging device captured in the video data, X-ray dose data generated by an X-ray dosimeter worn by one or more medical professionals 170, 180 captured in the video data 110, audio data, medical device user interface data 120s representing a user interaction with a user interface 190 of a medical device captured in the video data 110.

An example in which the sensor data includes log data 120₁ that is generated by a medical device 150, 160 captured in the video data 110 is illustrated on the left-hand side of Fig. 2. In this example, the medical device is a projection X-ray medical imaging system 150. The projection X-ray medical imaging system 150 is captured in the video data 110 that is generated by the camera 230. In general, the log data of a projection X-ray medical imaging system includes data from sensors such as orientation sensors that record the orientation of the C-arm that supports the X-ray source and X-ray detector, user interface sensors (e.g. switches such as a foot pedal that is used to initiate an imaging procedure) that are used to control the operation of the medical imaging system, and so forth. The log data of the projection X-ray medical imaging system 150 also includes timestamps corresponding to the time of acquisition of the sensor data by the respective sensors. The log data 120₁ of the projection X-ray medical imaging system 150 represents events that occur during a medical procedure. For instance, the C-arm orientation that is recorded by the C-arm orientation sensors, is initially in a parked position whilst the patient is prepared. Prior to guidewire insertion, the C-arm orientation is adjusted, often iteratively, to provide a desired imaging orientation. A change in the C-arm orientation from the parked position, as recorded by the C-arm orientation sensors, may therefore represent the event "preparation for guidewire insertion". During the event "guidewire insertion", the user interface sensors of the projection X-ray medical imaging system 150 may be used to acquire temporal sequences of X-ray images in order to navigate the guidewire within the vasculature. Thus, the event "guidewire insertion" may be detected via the user interface sensors that control image acquisition by the projection X-ray medical imaging system 150. At the end of the medical procedure, the C-arm orientation is returned to the parked position. Consequently, the corresponding change in the C-arm orientation back to the parked position, as recorded by the C-arm orientation sensors, may therefore represent the event "completion of intervention".

Another example in which the sensor data includes log data 120₂ that is generated by a medical device 150, 160 captured in the video data 110 is illustrated on the right-hand side of Fig. 2. In this example, the medical device is a contrast agent injector 160. The contrast agent injector 160 is captured in the video data 110 that is generated by the camera 230 illustrated in Fig. 2. A contrast agent injector, often referred-to simply as an "injector", is used to inject a contrast agent into a subject during a medical imaging procedure in order to enhance the visibility of the vasculature. The log data from an injector includes sensor data from sensors such as user interface sensors which are used to control the injection of the contrast agent in to the patient. The log data of the injector 160 also includes timestamps corresponding to the time of acquisition of the sensor data by the user interface sensors. Contrast agent is injected into the vasculature of a patient at specific times in a medical procedure. For instance, contrast agent is typically injected prior to the event "stent insertion" and wherein an angiogram is generated in order to perform a stent sizing procedure. A contrast agent may also be injected subsequent to stent expansion, i.e. during the event "stent verification", in order to confirm the shape of the expanded vessel. Log data from the injector 160 that includes sensor data from user interface sensors that record the times at which contrast agent is injected in the patient, may therefore represents the times of these events during a medical procedure.

In another example, the sensor data includes location data 120₃, 120₄ representing a location of one or more medical professionals 170, 180 captured in the video data 110. In the center of Fig. 2, an example is illustrated in which the location data 120₃ represents the location of a nurse 170. The nurse 170 is captured in the video data 110 that is generated by the camera 230. The location data 120₃ is provided by a body-mounted location-sensing tag 270 that is worn by the nurse 170. The location data also includes timestamps corresponding to the time of its acquisition by the location-sensing tag 270. The location-sensing tag may provide the location data 120₃ using various tracking techniques, including for example infrared signal tracking, or wireless RF signal tracking. The location data 120₃ represents the times of various events during a medical procedure that are supported by the nurse 170. For instance, the location data 120₃ may indicate a current location of the nurse 170 via with a granularity of a room in a medical facility (e.g. cath lab 1, corridor, etc.) in which the nurse 170 is located. The presence of the nurse in the cath lab is representative of such as the draping stage prior to an interventional procedure and wherein the patient 260 is being covered with sterile drapes. The location data 120₃ may indicate the current location of the nurse 170 with a granularity of a zone within a specific room in which the nurse is currently located. The zone may be representative of a specific event during the procedure. For instance, the zone may be representative of events such as the cleansing of an access site by the nurse, the preparation of a medical device such as a guidewire, and so forth.

By way of another example, on the left-hand side of Fig. 2, an example is illustrated wherein the location data 120₄ represents a location of a physician 180. In this example, the location data 120₄ is provided by the body-mounted location-sensing tag 280 that is worn by the physician 180. The location of the physician, e.g. within the cath lab, or within a zone within the cath lab, is similarly representative of various events during a medical procedure that are supported by the physician 180.

In another example, the sensor data includes motion data representing a motion of one or more medical professionals 170, 180 captured in the video data 110. This example is similar to the examples described above in which the location data 120₃, 120₄ is provided by body-mounted location-sensing tags 270, 280, with the difference that a body-mounted motion-sensing tag is used in place of a body-mounted location-sensing tag. In this example, the motion data may be provided by various motion sensors. For example, the motion data may be provided by an accelerometer, or by a gyroscope, or by a strain sensor, and so forth. The motion data represents the motion of the respective medical professional 170, 180, and is similarly indicative of the events that are performed by the wearer of the motion sensor. For instance, the event "attaching various vital sign monitoring equipment to the patient" may be represented by a characteristic set of motions of the body and the arms of a nurse, whereas the event "cleansing an access site in the arm" may be represented by another characteristic set of motions of the body and the arms of the nurse.

In another example, the sensor data includes medical image data 120s generated by a medical imaging device 150 captured in the video data 110. This example is illustrated on the left-hand side of Fig. 2, and wherein the sensor data 120s is generated by the projection X-ray medical imaging system 150. The projection X-ray medical imaging system 150 is captured in the video data 110. The position, and orientation of the projection X-ray medical imaging system 150 as captured in the video data 110 provides a link between the video data 110 and the medical image data 120s that is generated by the projection X-ray medical imaging system 150. The medical image data 120s also includes timestamps representing the times at which the medical image data 120s was acquired. The medical image data 120s represents the times of various events in a medical procedure during which medical images are generated. For instance, it represents the times at which a guidewire navigation, ballooning, or stenting, procedure takes place.

In another example, the sensor data includes X-ray dose data generated by an X-ray dosimeter worn by one or more medical professionals 170, 180 captured in the video data 110. In this example, a medical professional, such as the nurse 170, or the physician 180, wears an X-ray dosimeter. The medical professional is also captured in the video data 110. The dosimeter records the X-ray dose subjected to the medical professional, and the corresponding timestamps at which the dose was recorded. The X-ray dose data that is recorded by the dosimeter is therefore representative of the dose, and the corresponding time, at which events during the medical procedure that include X-ray imaging procedures, such as "guidewire insertion", "stent sizing" and "stent verification" are performed.

In another example, the sensor data includes audio data. The audio data may be generated by a microphone disposed in the vicinity of the medical professionals 170, 180. The microphone captures the conversations of the medical professionals, and also the timestamps of the conversations, and which is also representative of the stage of the procedure. Words that are used by the medical professionals 170, 180 may be extracted from the audio data using techniques such as natural language processing "NLP". The words that are used are also representative of the events that are performed during the medical procedure. The event may be determined by mapping occurrences of the words to one of a number of potential events using a decision tree, for example.

In another example, the sensor data includes medical device user interface data 120s representing a user interaction with a user interface 190 of a medical device captured in the video data 110. This example is illustrated in the central portion of Fig. 2, and wherein a user interface 190 of a guidewire manipulator (not illustrated in Fig. 2), is used to control the positioning of a guidewire (not illustrated in Fig 2) within the patient 260. The user interface 190 of the guidewire manipulator is captured in the video data. The user interface 90 may include various controls, e.g. buttons, a joystick, and so forth, which control the longitudinal, and also rotational, position of the guidewire within the vasculature of the subject via various effectors such as rollers, robotic fingers, and so forth. The medical device user interface data 120s includes timestamps representing the times at which the various controls were operated. In so doing, the medical device user interface data 120₅ represents the times of various events that relate to guidewire manipulation, were performed. By way of another example, the user interface may be the user interface of a medical imaging device, such as the projection X-ray medical imaging system 150 illustrated in Fig. 2. In this example, the user interface data 120s of the projection X-ray medical imaging system 150 represents the times of events such as the times of generation of X-ray images. In another example, the sensor data includes position sensor data 120₆ representing a position of a patient bed 250. The position of the patient bed is also indicative of various imaging-related events during a medical procedure. For instance, in a CT imaging procedure, the position of the patient bed may be extended into the bore of the CT imaging system during imaging, and in a withdrawn position prior to, and subsequent to imaging.

In general, the sensor data that is received in the operation S120 may be received via any form of data communication, including via wired, or wireless, or optical fiber communication. For instance, in the example illustrated in Fig. 2, the location data 120₃, 120₄ and the motion data 120₇ representing a motion of the vascular closure device 210, may be received via wireless communication, as depicted via the double-headed zigzag arrows associated with these items, and the video data 110 may be received via wired communication.

Referring now to the operation S130 illustrated in Fig. 1; in this operation, labels 130_{1..i} are assigned to the events captured in the video data 110 using the sensor data 120.

In general, the labels 130_{1..i} describe the events that are captured in the video data 110. In general, the labels may be assigned to the events that are captured in the video data, in order to provide annotated data that may be used to train a machine learning model to monitor the events that are captured in subsequently-generated video data during a medical procedure. Some examples of the assignment of labels to the events captured in the video data are described below.

In one example, the events comprise actions performed by one or more medical professionals 170, 180, or actions performed using one or more objects 210, and the sensor data 120₃, 120₄, 120₇ comprises a label identifying at least one of the one or more medical professionals 170, 180, or at least one of the one or more objects 210, respectively. In this example, the operation of assigning S130 labels 130_{1..i} to the events captured in the video data 110 using the sensor data 120, comprises assigning the label identifying the at least one medical professional 170, 180 to the corresponding medical professional captured in the video data 110, or assigning the label identifying the at least one object 210 to the corresponding object captured in the video data 110, respectively.

By assigning labels representing a medical professional, or an object, to the events captured in the video data, annotated data is provided that may be used to train a machine learning model to monitor events that are performed by a medical professional, or using an object, that is captured in subsequently-generated video data during a medical procedure.

This example is useful in distinguishing between different medical professionals, or different objects, in situations in which the events may be performed by different medical professionals, or in situations in which the events may be performed using different objects. For instance, in the example illustrated in Fig. 2, two medical professionals, the nurse 170 and the physician 180, are illustrated. When a person enters the room, this event may be detected in the video data 110 as the event "person enters cath lab". However, it may not be possible to determine from the video data alone whether the person that enters the cath lab is the nurse 170, or the physician 180. However, if both the nurse 170 and the physician 180 wear a tag that provides location data representing a location of the medical professional captured in the video data, and the location data also comprises a label identifying the respective medical professional 170, 180, then label "nurse enters cath lab" may be assigned to an event captured in the video data 110 when it is the nurse that enters the cath lab, and the label "physician enters cath lab" may be assigned to an event captured in the video data 110 when it is the physician that enters the cath lab.

Similarly, in situations in which the events that are captured in the video data 110 may be performed using different objects it is useful to distinguish between the different objects in order to correctly assign the objects to the events. For instance, in the lower portion of the example illustrated in Fig. 2, a vascular closure device 210 is illustrated. A motion sensor 290 may be attached to the vascular closure device 210 to provide motion data 120₇ representing a motion of the vascular closure device 210. A motion sensor may similarly be coupled to another medical device, such as an anesthesia injection device and similarly provide motion data representing a motion of the anesthesia injection device. When one of the medical devices is picked-up for use, this may be detected in the video data 110 as the event "medical device has been picked-up". However, it may not be possible to determine from the video data alone whether the medical device that has been picked-up is the vascular closure device 210 or the anesthesia injection device. However, if both the vascular closure device 210 and the anesthesia injection device include a tag that provides motion data representing a motion of the respective device captured in the video data, and the motion data also comprises a label identifying the respective device, then labels 130_{1..i} such as "vascular closure device in-use" and "the anesthesia injection device in-use" may be correctly assigned to the events captured in the video data 110.

In some examples, the sensor data includes sufficient information for the event to be determined directly from the sensor data. In these examples, the labels may be assigned to the events directly from the sensor data. In these examples, the sensor data may be described as a raw waveform, or a raw value, representing a measured parameter. For instance, sensor data that is provided in the form of user interface data 120s representing a user interaction with a user interface of a medical imaging system captured in the video data, directly represents a user command to initiate an imaging procedure. In this case, the event "imaging procedure initiated" is directly derivable from the sensor data. Likewise, sensor data that sensor data that is provided in the form of location data 120₃ representing a location of a nurse captured in the video data 110 directly represents the event "nurse enters cath lab". In this case, the event "nurse enters cath lab" is directly derivable from the raw sensor data.

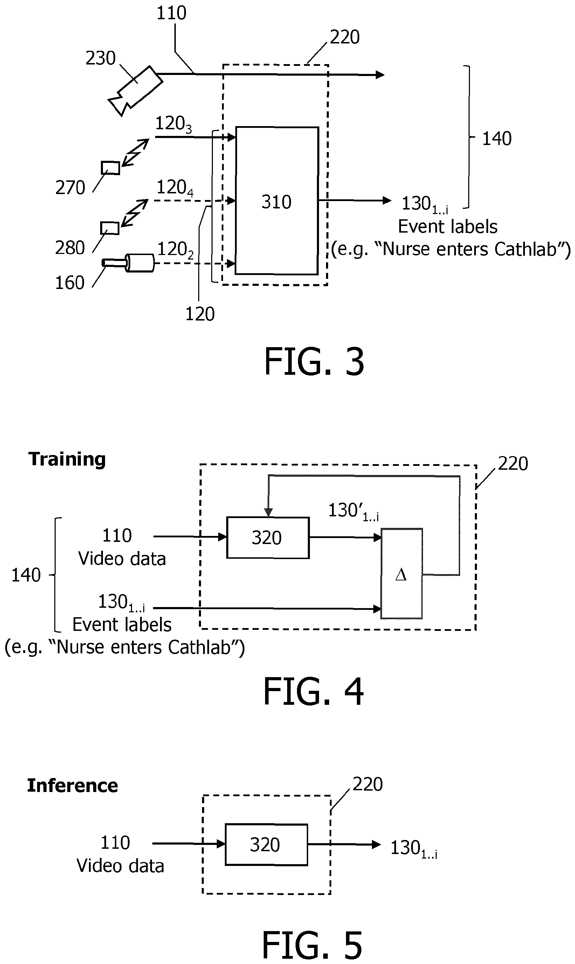

In other examples, however, the operation of assigning S130 labels 130_{1..i} to the events captured in the video data 110 using the sensor data 120, includes extracting, from the sensor data 120, event labels describing the events, and assigning the extracted event labels to the corresponding events captured in the video data 110. The event labels may be extracted from the sensor data using an algorithm for example. An example in which event labels are extracted from sensor data using an algorithm is illustrated in Fig. 3, which is a schematic diagram illustrating an example of various elements of a computer-implemented method of providing annotated data, in accordance with some aspects of the present disclosure. In the example illustrated in Fig. 3, an algorithm 310 is used to extract event labels 130_{1..i} from the location data 120₃ representing the location of the nurse 170 illustrated in Fig. 2, from the location data 120₄ representing the location of the physician 180, and from the log data is generated by the controller 190 of the guidewire (not illustrated in Fig. 2). In general, one or more types of data may be inputted into one or more algorithms in order to determine the event labels 130_{1..i}. For instance, in some examples, a separate algorithm is used to determine the event labels of each of sensor data 120₂, 120₃, and 120₄. In other examples, multiple types of sensor data 120 are inputted into a single algorithm such that the algorithm may use correspondences between the different types of sensor data to improve the accuracy of the outputted event labels. In general, an algorithm may extract the events based on patterns in the sensor data 120. For instance, patterns in motion data representing the arms of a physician may represent events that are performed by a physician such as guidewire manipulation, contrast agent injection, vascular closure, and so forth. Similarly, patterns in the location data 120₃ of a nurse may be used to determine a specific event in which the nurse is engaged such as "draping" transfer of patient to recovery room" and so forth. Similarly, patterns in the motion data 120₇ representing a motion of a vascular closure device 210 may be indicative of its use in the event "closure of access site". The algorithm may be implemented using various machine learning techniques, including deep learning techniques and neural networks.

In another example, the medical procedure comprises an X-ray imaging procedure performed by a medical professional 170, 180. The events comprise X-ray exposure events experienced by the medical professional 170, 180 during the X-ray imaging procedure, and the sensor data 120 comprises X-ray dose data generated by an X-ray dosimeter worn by the medical professional 170, 180. In this example, the operation of assigning S130 labels 130_{1..i} to the events captured in the video data 110 using the sensor data 120, comprises assigning to the medical professional 170, 180 captured in the video data 110, dose labels representing the X-ray dose experienced by the medical professional 170, 180 during the X-ray imaging procedure.

In this example, the X-ray dose data comprises an indication of an amount of dose experienced by the medical professional 170, 180. The X-ray dose data also includes timestamps indicating the time of the dose. In this example, the dose data is assigned to the medical professional that is captured in the video data. By assigning the dose data to the medical professional captured in the video data, annotated video data is provided that may be used to train a machine learning model to monitor the amount of dose experienced by the medical professional from subsequently-generated video data. The trained machine learning model may be used to monitor the X-ray dose experienced by the medical professional without the need for the medical professional to wear an X-ray dosimeter.

In another example, the video data 110 comprises a temporal sequence of X-ray images. The events comprise actions performed using one or more interventional devices, and the sensor data 120 comprises log data generated by a controller 190 of at least one of the one or more interventional devices. In this example, the operation of assigning labels 130_{1..i} to the events captured in the video data 110 using the sensor data 120, comprises extracting, from the log data, event labels describing the events, and assigning the extracted event labels to the corresponding events captured in the temporal sequence of X-ray images.

In this example, the temporal sequence of images may be fluoroscopy images, for example. Fluoroscopy images are often generated during interventional procedures in order to perform tasks such as interventional device navigation and deployment. Fluoroscopy images are typically generated in real-time. Fluoroscopy images typically use an X-ray dose per image that is relatively lower than that incurred by a single diagnostic X-ray image. The interventional device in this example may be an injector, a guidewire, or a stent deployment device, for example. The log data of the controller of the injector, for example, is indicative of e.g. the amounts and the times at which contrast agent is injected into the vasculature. An example of a label that may be applied to an event captured in the fluoroscopy images in this example is an amount of contrast agent that is injected into the patient. This amount of injected contrast agent is recorded in the log data of the controller of the injector. The fluoroscopy images may then be labelled with a label such as "injected amount of contrast agent is X ml". By assigning extracted event labels such as this to the corresponding events captured in the temporal sequence of X-ray images, annotated video data in the form of a temporal sequence of X-ray images is provided which may be used to train a machine learning model to monitor such events from subsequently-generated X-ray image data during a medical procedure.

Referring now to the operation S140 illustrated in Fig. 1; in this operation, the video data 110 and the labels 130_{1..i} are outputted to provide the annotated data 140.

The annotated data 140 may be outputted in various ways, including to a computer-readable storage medium, or to the Internet, or to the Cloud, or to a display device such as to the display 240 illustrated in Fig. 2, or to a virtual/ augmented reality display device, or to a printer, and so forth. The annotated data may be used to train a machine learning model to monitor such events from subsequently-generated video data during a medical procedure, as described in the examples below.

As mentioned, above, the method described above with reference to Fig. 1 may also be implemented by one or more processors 220 of the 200 illustrated in Fig. 2. Thus, in one example, a system for providing annotated data, is provided. The system comprises one or more processors 220 configured to:
receive S110 video data 110, the video data capturing events during a plurality of instances of a medical procedure;
receive S120 sensor data 120, the sensor data representing the events during the instances of the medical procedure, and wherein the sensor data 120 for each instance of the medical procedure is generated contemporaneously with the video data 110 for each instance of the medical procedure;
assign S130 labels 130_{1..i} to the events captured in the video data 110 using the sensor data 120; and
output S140 the video data 110 and the labels 130_{1..i} to provide the annotated data 140.

As also mentioned, above, in one example, the annotated data 140 described above is used to train a machine learning model to monitor events during a medical procedure.

In this example, a computer-implemented method of training a machine learning model 320 to monitor events during a medical procedure, includes:
receiving the annotated data 140;
inputting the video data 110 into the machine learning model 320; and
training the machine learning model 320 to predict, based on the video data 110, the events captured in the video data, using the corresponding labels 130_{1..i}.

This example is illustrated in Fig. 4, which is a schematic diagram illustrating an example of various elements of a computer-implemented method of training a machine learning model 320 to monitor events during a medical procedure, in accordance with some aspects of the present disclosure. On the left-hand side of Fig. 4, an example of annotated data 140 is shown in which the annotated data 140 includes the video data 110 and the labels 130i i. The video data 110 is inputted into the machine learning model 320. The machine learning model 320 illustrated in Fig. 4 may be implemented by one or more processors 220. The machine learning model 320 may be implemented by the one or more processors 220 using various machine learning techniques, including deep learning techniques and neural networks. When provided by a neural network, the neural network may have various architectures. For instance, the neural network may be provided by a U-Net, a vision transformer architecture, a recurrent neural network "RNN", an encoder/ decoder architecture, and so forth.

In general, the training of the machine learning model 320 involves inputting the training data, in this case the video data 110, into the machine learning model, and iteratively adjusting the model's parameters until the trained machine learning model provides an accurate output. In the example illustrated in Fig. 4, the video data 110 is inputted into the machine learning model 320, and in response to the inputting, the machine learning model outputs the predicted labels 130'_{1..i}. The predicted labels 130'_{1..i} describe the events captured in the video data 110. A deviation, Δ, between the predicted labels 130'_{1..i} and the intended output of the machine learning model, i.e. the labels 130_{1..i} from the annotated data 140, is used to adjust the parameters of the machine learning model 320.

In general, the machine learning model 320 may be trained to predict the events captured in the video data 110 by:
for each of a plurality of instances of the medical procedure:
inputting the video data 110 for the instance from the annotated data into the machine learning model;
predicting one or more events 130'_{1..i}, using the machine learning model, in response to the inputting; and
adjusting parameters of the machine learning model based on a difference, Δ, between the one or more events 130'_{1..i} predicted by the machine learning model, and the corresponding one or more events 130_{1..i} for the instance of the medical procedure from the annotated data; and
repeating the inputting, the predicting, and the adjusting, until a stopping criterion is met.

In the example mentioned above in which the machine learning model 320 is a neural network, the parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem, including gradient descent, Quasi-Newton methods, and so forth. Various algorithms have been developed to implement these methods and their variants, including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers". These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

The training of a neural network is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The trained machine learning model may then be used to monitor events during a medical procedure by predicting, or inferring, the events from video data that is inputted into the model. Thus, in one example, a computer-implemented method of monitoring events during a medical procedure, includes:
receiving video data 110 capturing the events during the medical procedure;
inputting the video data 110 capturing the events during the medical procedure, into the trained machine learning model 320;
predicting, using the trained machine learning model 320, and based on the inputted video data 110, one or more events captured in the video data 110; and
outputting the predicted one or more events.

This example is described with reference to Fig. 5, which is a schematic diagram illustrating an example of various elements of a computer-implemented method of monitoring events during a medical procedure, in accordance with some aspects of the present disclosure. As illustrated in Fig. 5, the trained machine learning model 320 is implemented by one or more processors 220. As illustrated on the left-hand side of Fig. 5, video data 110 is inputted into the trained machine learning model 320, and in response, the trained machine learning model 320 outputs the predicted events 130_{1..i}. The predicted events 130_{1..i} may be outputted in various ways, including to a computer-readable storage medium, or to the Internet, or to the Cloud, or to a display device, or to a virtual/ augmented reality display device, or to a printer, and so forth.

In another example, a system 400 that corresponds to the above-described computer-implemented method of monitoring events during a medical procedure, is provided. In this example, a system 400 for monitoring events during a medical procedure includes one or more processors 220 configured to:
receive video data 110 capturing the events during the medical procedure;
input the video data 110 into a trained machine learning model 320; and
predict, using the trained machine learning model 320, and based on the inputted video data 110, the one or more events captured in the video data 110; and
output the predicted one or more events; and
wherein the trained machine learning model 320 is trained to predict the one or more events captured in the video data 110 using annotated data 140, the annotated data comprising video data 110 capturing events during a plurality of instances of the medical procedure, and ground truth labels 130_{1..i} representing the events captured in the video data 110, and wherein the ground truth labels 130_{1..i} are assigned to the events captured in the video data 110 using sensor data 120 representing the events, and wherein the sensor data 120 is generated contemporaneously with the video data 110.

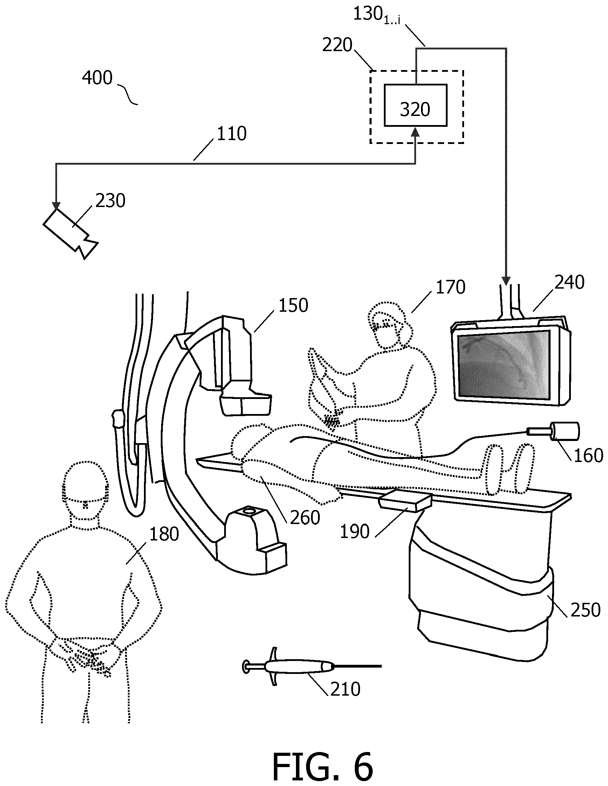

This example is described with reference to Fig. 6, which is a schematic diagram illustrating an example of a system 400 for monitoring events during a medical procedure, in accordance with some aspects of the present disclosure. In the example illustrated in Fig. 6, the camera 230 generates video data 110 that captures events during the medical procedure. The medical procedure illustrated in Fig. 6 may in general be any medical procedure. The medical procedure may be a cardiac procedure such as an angioplasty procedure, as described in relation to the provision of the annotated data 140. The events that are captured in the video data 110 may be events such as a medical professional (e.g. the nurse 170, or the physician 180, entering the cath lab); a patient being brought into the cath lab; the attaching of various vital sign monitoring equipment to the patient; and so forth, as described above with reference to Fig. 2. As illustrated in Fig. 6, the video data 110 is inputted into the trained machine learning model 320. The trained machine learning model 320 may be implemented by one or more processors 220. In response to the inputting, the trained machine learning model 320 predicts the events 130_{1..i} that are captured in the video data 110. The predicted events 130_{1..i} may be outputted in various ways, including to a computer-readable storage medium, or to the Internet, or to the Cloud, or to a display device, or to a virtual/ augmented reality display device, or to a printer, and so forth. In the example illustrated in Fig. 6, the events 130_{1..i} are outputted to the display device 240. The events may be outputted in various ways, such as in the form of text, or as an icon, for example.

In another example, a computer program product is provided. The computer program product corresponds to the above-described computer-implemented method of monitoring events during a medical procedure, and which was described with reference to Fig. 5. The computer program product comprises instructions which when executed by one or more processors, cause the one or more processors to carry out a method of monitoring events during a medical procedure. The method comprises:
receiving video data 110 capturing the events during the medical procedure;
inputting the video data 110 capturing the events during the medical procedure, into the trained machine learning model 320;
predicting, using the trained machine learning model 320, and based on the inputted video data 110, one or more events captured in the video data 110; and
outputting the predicted one or more events.

In general, the events that are predicted in accordance with the method described with reference to Fig. 5, may be predicted without inputting into the trained machine learning model, sensor data 120 representing the events during the medical procedure. Thus, at inference, it obviates the need to acquire the sensor data 120 (described in relation to Fig. 2) that was used to annotate the video data 110. This simplifies the implementation of the method and the corresponding system 400.

In one example, the events that are predicted in accordance with the method described with reference to Fig. 5 are used to provide guidance for a medical procedure. In this example, the computer-implemented method described with reference to Fig. 5 includes:
determining, based on the predicted one or more events, a current stage in a medical procedure schedule; and
outputting guidance for the medical procedure based on a comparison between the current stage in the medical procedure schedule and an expected stage of the medical procedure schedule.

The medical procedure schedule includes a sequence of events, such as those described above for the example angioplasty procedure, together with an expected time, or duration, of the event. The expected times, or durations, may be set in accordance with a protocol for a medical facility. The current stage in the medical procedure schedule may be determined by comparing the predicted events with scheduled events in the medical procedure schedule. Various types of guidance may be outputted in accordance with this example. For instance, in one example, guidance can be outputted in the form of an indication of the performance of the current event, or the current procedure in relation to an expected duration, or time. A traffic light indication may be provided to indicate the performance in relation to the medical procedure schedule, for example. In another example, the guidance may include recommendations to expedite a current event in the procedure. For example, the guidance may include a recommendation to request additional medical professionals, or additional equipment, to expedite the medical procedure.

In one example, further sensor data is acquired at the time of performing inference with the trained machine learning model. The further sensor data is used to further train the trained machine learning mode l320. In this example, the computer-implemented method described with reference to Fig. 5 includes:
receiving further sensor data 120 representing the events during the medical procedure, wherein the further sensor data 120 is generated contemporaneously with the video data 110 that is inputted into the trained machine learning model 320;
assigning further labels 130_{1..i} to the events captured in the video data 110 that is inputted into the trained machine learning model 320, using the further sensor data 120; and
further training the machine learning model 320 to predict the events captured in the video data 110, using the video data 110 that is inputted into the trained machine learning model 320, and the corresponding further labels 130_{1..i}.

Performing further training of the machine learning model using the sensor data that is acquired at inference time enables the trained model to take account of changes in the environment, such as medical professionals wearing different clothing, or the presence of additional medical professionals, or additional objects in the cath lab. This in-turn improves the accuracy of the predicted events that are predicted by the trained machine learning model.

In another example, further sensor data is acquired at the time of performing inference with the trained machine learning model. The further sensor data is used to determine the events that are captured by the video data in order to provide a comparison with the events that are predicted by the trained machine learning model 320. In this example, the computer-implemented method described with reference to Fig. 5 includes:
receiving further sensor data 120 representing the events during the medical procedure, wherein the further sensor data 120 is generated contemporaneously with the video data 110 that is inputted into the trained machine learning model;
assigning labels 130_{1..i} to the events captured in the video data 110 that is inputted into the trained machine learning model, using the further sensor data 120;
selecting as the correct one or more events, either the one or more events predicted by the trained machine learning model, or the assigned label; and
wherein the outputting the predicted one or more events comprises outputting the one or more events selected as the correct one or more events.

In this example, the labels 130_{1..i} may be assigned to the events captured in the video data 110 that is inputted into the trained machine learning model, using the algorithm 310 that was described above with reference to Fig. 3. The algorithm provides a second label for the events captured in the video data 110. The correct event is then selected from the label that is assigned by the algorithm, and the event that is predicted by the trained machine learning model. The correct event may be selected by using a regression-based decision model to identify the most likely event to have occurred. A Nominal Logistic Regression model may be used, for example. Selecting the event in accordance with this example may improve the reliability of the event that is determined to be correct.

As mentioned above, in a second aspect of the present disclosure, a computer-implemented method of monitoring events captured in one or medical images 510 generated by a medical imaging system 150 during a medical imaging procedure, is provided. This method comprises:
receiving S510 medical imaging system log file data 520 generated by the medical imaging system 150 during the medical imaging procedure;
inputting S520 the medical imaging system log file data 520 into a trained machine learning model 530, the trained machine learning model being trained to predict, from the log file data 520, one or more events 130_{1..i} captured in the one or more medical images 510 generated during the medical procedure;
predicting S530, using the trained machine learning model 530, and based on the inputted medical imaging system log file data 520, one or more events 130_{1..i} captured in the one or more medical images 510 generated during the medical procedure; and
outputting S540 the predicted one or more events 130_{1..i}; and
wherein the machine learning model 530 is trained to predict the one or more events 130_{1..i} captured in the one or more medical images 510 generated during the medical procedure using annotated data, the annotated data comprising medical imaging system log file data generated during a plurality of instances of the medical imaging procedure, and ground truth event labels representing one or more events 130_{1..i} captured in one or more medical images 510 generated during the instances of the medical procedure, and wherein the ground truth event labels are extracted from the one or more medical images 510.

In this method, a machine learning model is trained to predict events captured in medical images generated during a medical procedure. Examples of events that may be predicted in accordance with this second aspect include "catheter positioned in treatment site" "angioplasty balloon inflation in progress", "stent expansion initiated", and so forth. The events are predicted from medical imaging system log file data. Thus, at inference, the events are predicted without the need to analyze the medical images themselves. This circumvents privacy issues that might otherwise be encountered by determining the events by analyzing the medical images. The machine learning model that is used in this method is trained to predict the events using annotated data that comprises medical imaging system log file data that is generated during a plurality of instances of the medical imaging procedure, and ground truth event labels. The ground truth event labels are extracted from the one or more medical images that are generated during the instances of the medical procedure. This is in contrast to the method described in accordance with the first aspect and wherein the annotated data included video data that was labelled with event labels that were assigned using sensor data. Since in this method the event labels are extracted from medical images, accurate event labels are provided, and the burden of manually labelling the log file data is also reduced.

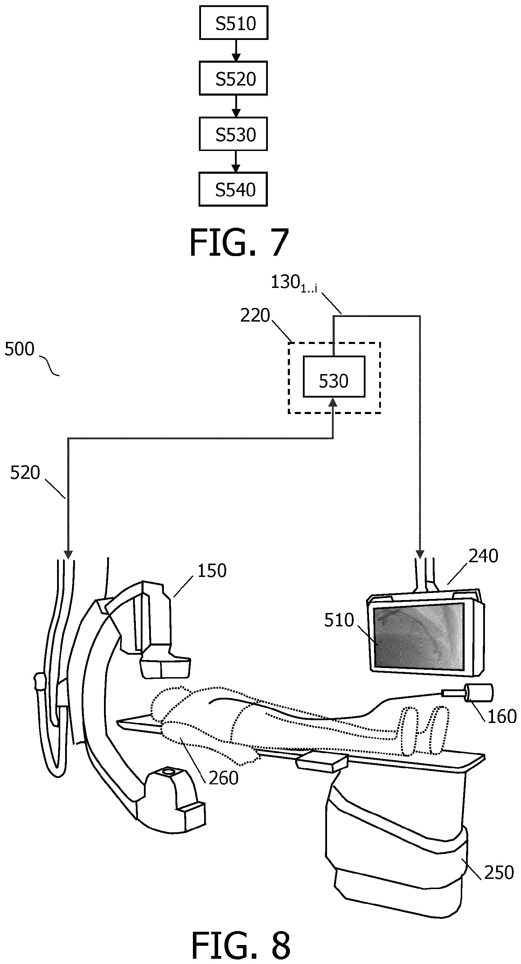

This method is described with reference to Fig. 7 and Fig. 8. Fig. 7 is a flowchart illustrating an example of a computer-implemented method of monitoring events captured in one or medical images 510 generated by a medical imaging system 150 during a medical imaging procedure, in accordance with some aspects of the present disclosure. Fig. 8 is a schematic diagram illustrating an example of a system 500 for monitoring events captured in one or medical images 510 generated by a medical imaging system 150 during a medical imaging procedure, in accordance with some aspects of the present disclosure. It is noted that operations that are described in relation to the method illustrated in Fig. 7, may also be performed by the one or more processors 220 of the system 500 illustrated in Fig. 8. Likewise, operations that are described as being performed by the one or more processors 220 of the system 500 illustrated in Fig. 8, may also be performed in the method described with reference to Fig. 7.

The method illustrated in Fig. 7 may be used in various medical imaging procedures. For instance, it may be used in diagnostic procedures, and also interventional procedures. In general, the medical imaging procedure may be used to image any part of the anatomy. For instance, the medical imaging procedure may involve imaging of the heart, or the brain, or the chest, or a limb, and so forth. The method may be used in the angioplasty procedure described above, for example.

In this method, the medical imaging system log file data 520 that is received in the operation S510 may include information relating to commands executed by the medical imaging system. The medical imaging system log file data 520 may also include timestamps corresponding to the times at which the commands were executed. For instance, in the example illustrated in Fig. 8 and wherein the medical imaging system is a projection X-ray medical imaging system 150, the log file data may include data relating to image acquisition settings such as the C-arm orientation, the exposure time, the X-ray beam energy, the X-ray dose, the type of imaging mode (e.g. single shot versus fluoroscopy) and so forth, together with their corresponding timestamps .

At inference, the medical imaging system log file data 520 is inputted into the trained machine learning model 530, and the trained machine learning model 530 predicts the events 130_{1..i} that are captured in the medical image(s) 510 generated during the medical procedure. The predicted events 130_{1..i} may be outputted in various ways, including to a display device such as to the display 240 illustrated in Fig. 8, or to a virtual/ augmented reality display device, or to a printer, or to a computer-readable storage medium, or to the Internet, or to the Cloud, and so forth.

The machine learning model 530 may be provided, and also trained, in a similar manner to the machine learning model 320 described above with reference to Fig. 4 and Fig. 5. The machine learning model 530 may be implemented by one or more processors 220, as illustrated in Fig. 8. In this machine learning model 530, however, different annotated data is used to train the model. In this method, the annotated data includes medical imaging system log file data that is generated during a plurality of instances of the medical imaging procedure, and ground truth event labels representing one or more events 130_{1..i} captured in one or more medical images 510 generated during the instances of the medical procedure. The ground truth event labels are extracted from the one or more medical images 510. The ground truth event labels may be extracted from the medical images 510 using automated techniques such as machine vision techniques, and a trained machine learning model. The annotated data may represent some tens, or hundreds, or more, medical procedures. In general, at inference, the events that are predicted in accordance with the method described with reference to Fig. 7, and in accordance with the system illustrated in Fig. 8, are predicted without inputting into the trained machine learning model 530, medical images that are generated during the medical procedure. This circumvents privacy issues that might otherwise be encountered by determining the events by analyzing the medical images.

As mentioned above, this method may also be implemented in the form of the system 500 illustrated in Fig. 8. Thus, in one example, a system for monitoring events captured in one or medical images 510 generated by a medical imaging system 150 during a medical imaging procedure, is provided. The system comprises one or more processors 220 configured to:
receive S510 medical imaging system log file data 520 generated by the medical imaging system 150 during the medical imaging procedure;
input S520 the medical imaging system log file data 520 into a trained machine learning model 530, the trained machine learning model being trained to predict, from the log file data 520, one or more events 130_{1..i} captured in the one or more medical images 510 generated during the medical procedure;
predict S530, using the trained machine learning model 530, and based on the inputted medical imaging system log file data 520, one or more events 130_{1..i} captured in the one or more medical images 510 generated during the medical procedure; and
output S540 the predicted one or more events 130_{1..i}; and
wherein the machine learning model 530 is trained to predict the one or more events 130_{1..i} captured in the one or more medical images 510 generated during the medical procedure using annotated data, the annotated data comprising medical imaging system log file data generated during a plurality of instances of the medical imaging procedure, and ground truth event labels representing one or more events 130_{1..i} captured in one or more medical images 510 generated during the instances of the medical procedure, and wherein the ground truth event labels are extracted from the one or more medical images 510.

It is noted that the systems 200, 400, and 500 described above with reference to Fig. 2, Fig. 6 and Fig. 8 respectively may also include one or more of: a medical imaging system, such as for example the projection X-ray medical imaging system 150 illustrated in these Figures, a camera 230 for providing the video data 110, an injector 160 for injecting a contrast agent into a patient; a display device, such as the display 240 illustrated in these Figures; a patient bed 250; and a user input device (not illustrated) configured to receive user input in relation to the operations performed by the one or more processors 220, such as a keyboard, a mouse, a touchscreen, and so forth.

The above examples are to be understood as illustrative of the present disclosure, and not restrictive. Further examples are also contemplated. For instance, the examples described in relation to a computer-implemented method, may also be provided by a corresponding computer program product, or by a corresponding computer-readable storage medium, or by a corresponding system, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing annotated data, the method comprising: receiving (S 110) video data (110), the video data capturing events during a plurality of instances of a medical procedure;
receiving (S120) sensor data (120), the sensor data representing the events during the instances of the medical procedure, and wherein the sensor data (120) for each instance of the medical procedure is generated contemporaneously with the video data (110) for each instance of the medical procedure;
assigning (S130) labels (130_{1..i}) to the events captured in the video data (110) using the sensor data (120); and
outputting (S140) the video data (110) and the labels (130_{1..i}) to provide the annotated data (140).

2. The computer-implemented method according to claim 1 wherein the sensor data (120) comprises one or more of: log data (120_{1,} 120₂) generated by a medical device (150, 160) captured in the video data (110), location data (120₃, 120₄) representing a location of one or more medical professionals (170, 180) captured in the video data (110), motion data representing a motion of one or more medical professionals (170, 180) captured in the video data, medical image data (120s) generated by a medical imaging device captured in the video data, X-ray dose data generated by an X-ray dosimeter worn by one or more medical professionals (170, 180) captured in the video data (110), audio data, medical device user interface data (120s) representing a user interaction with a user interface (190) of a medical device captured in the video data (110).

3. The computer-implemented method according to claim 1 or claim 2, wherein the events comprise actions performed by one or more medical professionals (170, 180), or actions performed using one or more objects (210), and wherein the sensor data (120₃, 120₄, 120₇) comprises a label identifying at least one of the one or more medical professionals (170, 180), or at least one of the one or more objects (210), respectively; and
wherein the assigning (S130) labels (130_{1..i}) to the events captured in the video data (110) using the sensor data (120), comprises assigning the label identifying the at least one medical professional (170, 180) to the corresponding medical professional captured in the video data (110), or assigning the label identifying the at least one object (210) to the corresponding object captured in the video data (110), respectively.

4. The computer-implemented method according to any one of claims 1-3, wherein the assigning (S130) labels (130_{1..i}) to the events captured in the video data (110) using the sensor data (120), comprises extracting, from the sensor data (120), event labels describing the events, and assigning the extracted event labels to the corresponding events captured in the video data (110).

5. The computer-implemented method according to any one of claims 1-4, wherein the medical procedure comprises an X-ray imaging procedure performed by a medical professional (170, 180), wherein the events comprise X-ray exposure events experienced by the medical professional (170, 180) during the X-ray imaging procedure, and wherein the sensor data (120) comprises X-ray dose data generated by an X-ray dosimeter worn by the medical professional (170, 180); and
wherein the assigning (S130) labels (130_{1..i}) to the events captured in the video data (110) using the sensor data (120), comprises assigning to the medical professional (170, 180) captured in the video data (110), dose labels representing the X-ray dose experienced by the medical professional (170, 180) during the X-ray imaging procedure.

6. The computer-implemented method according to claim 1, wherein the video data (110) comprises a temporal sequence of X-ray images, wherein the events comprise actions performed using one or more interventional devices, and wherein the sensor data (120) comprises log data generated by a controller (190) of at least one of the one or more interventional devices;
wherein the assigning labels (130_{1..i}) to the events captured in the video data (110) using the sensor data (120), comprises extracting, from the log data, event labels describing the events, and assigning the extracted event labels to the corresponding events captured in the temporal sequence of X-ray images.

7. A computer-implemented method of training a machine learning model (320) to monitor events during a medical procedure, the method comprising:
receiving the annotated data (140) according to any one of claims 1-6;
inputting the video data (110) into the machine learning model (320); and
training the machine learning model (320) to predict, based on the video data (110), the events captured in the video data, using the corresponding labels (130_{1..i}).

8. A computer-implemented method of monitoring events during a medical procedure, the method comprising:
receiving video data (110) capturing the events during the medical procedure;
inputting the video data (110) capturing the events during the medical procedure, into the trained machine learning model (320) according to claim 7;
predicting, using the trained machine learning model (320), and based on the inputted video data (110), one or more events captured in the video data (110); and
outputting the predicted one or more events.

9. The computer-implemented method according to claim 8, wherein the predicting is performed without inputting into the trained machine learning model, sensor data (120) representing the events during the medical procedure.

10. The computer-implemented method according to claim 8 or claim 9, further comprising: determining, based on the predicted one or more events, a current stage in a medical procedure schedule; and
outputting guidance for the medical procedure based on a comparison between the current stage in the medical procedure schedule and an expected stage of the medical procedure schedule.

11. The computer-implemented method according to any one of claims 8 - 10, further comprising:
receiving further sensor data (120) representing the events during the medical procedure, wherein the further sensor data (120) is generated contemporaneously with the video data (110) that is inputted into the trained machine learning model (320);
assigning further labels (130_{1..i}) to the events captured in the video data (110) that is inputted into the trained machine learning model (320), using the further sensor data (120); and
further training the machine learning model (320) to predict the events captured in the video data (110), using the video data (110) that is inputted into the trained machine learning model (320), and the corresponding further labels (130_{1..i}).

12. The computer-implemented method according to any one of claims 8 - 11, further comprising:
receiving further sensor data (120) representing the events during the medical procedure, wherein the further sensor data (120) is generated contemporaneously with the video data (110) that is inputted into the trained machine learning model;
assigning labels (130_{1..i}) to the events captured in the video data (110) that is inputted into the trained machine learning model, using the further sensor data (120);
selecting as the correct one or more events, either the one or more events predicted by the trained machine learning model, or the assigned label; and
wherein the outputting the predicted one or more events comprises outputting the one or more events selected as the correct one or more events.

13. A computer program product comprising instructions which when executed by one or more processors, cause the one or more processors to carry out the method according to any one of claims 1 - 12.

14. A system (400) for monitoring events during a medical procedure, the system comprising one or more processors (220) configured to:
receive video data (110) capturing the events during the medical procedure;
input the video data (110) into a trained machine learning model (320); and
predict, using the trained machine learning model (320), and based on the inputted video data (110), the one or more events captured in the video data (110); and
output the predicted one or more events; and
wherein the trained machine learning model (320) is trained to predict the one or more events captured in the video data (110) using annotated data (140), the annotated data comprising video data (110) capturing events during a plurality of instances of the medical procedure, and ground truth labels (130_{1..i}) representing the events captured in the video data (110), and wherein the ground truth labels (130_{1..i}) are assigned to the events captured in the video data (110) using sensor data (120) representing the events, and wherein the sensor data (120) is generated contemporaneously with the video data (110).

15. A computer-implemented method of monitoring events captured in one or medical images (510) generated by a medical imaging system (150) during a medical imaging procedure, the method comprising:
receiving (S510) medical imaging system log file data (520) generated by the medical imaging system (150) during the medical imaging procedure;
inputting (S520) the medical imaging system log file data (520) into a trained machine learning model (530), the trained machine learning model being trained to predict, from the log file data (520), one or more events (130_{1..i}) captured in the one or more medical images (510) generated during the medical procedure;
predicting (S530), using the trained machine learning model (530), and based on the inputted medical imaging system log file data (520), one or more events (130_{1..i}) captured in the one or more medical images (510) generated during the medical procedure; and
outputting (S540) the predicted one or more events (130_{1..i}); and
wherein the machine learning model (530) is trained to predict the one or more events (130_{1..i}) captured in the one or more medical images (510) generated during the medical procedure using annotated data, the annotated data comprising medical imaging system log file data generated during a plurality of instances of the medical imaging procedure, and ground truth event labels representing one or more events (130i i) captured in one or more medical images (510) generated during the instances of the medical procedure, and wherein the ground truth event labels are extracted from the one or more medical images (510).
